Europäisches Patentamt

European Patent Office

Office européen des brevets

(11) Publication number: **0 480 310 A2**

# EUROPEAN PATENT APPLICATION

(21) Application number: **91116887.0**

(22) Date of filing: **03.10.91**

(51) Int. Cl.5: **C12N 15/82**, C12N 15/40, A01H 5/00

(30) Priority: **09.10.90 JP 271165/90**

(43) Date of publication of application:
**15.04.92 Bulletin 92/16**

(84) Designated Contracting States:
**AT BE CH DE DK ES FR GB GR IT LI LU NL SE**

(71) Applicant: **TAKEDA CHEMICAL INDUSTRIES, LTD.**
**1-1, Doshomachi 4-chome**
**Chuo-ku, OSAKA(JP)**

(72) Inventor: **Komiya, Takeya**
**7-43, Minamisakurazuka 1-chome**
**Toyonaka, Osaka 560(JP)**
Inventor: **Ozaki, Kazuo**
**4-104, 1-14, Koaza-saihoji, Aza-enmyoji**
**Ohyamazaki-cho, Otokuni-gun, Kyoto 618(JP)**
Inventor: **Yoshida, Shigeru**
**c/o Mr. Kiyoo Morita, 2-30, Shibaharacho**
**4-chome**
**Toyonaka, Osaka 560(JP)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

(54) **Virus-resistant tomato plants.**

(57) The present invention provides a virus-resistant tomato plant as transformed with the coat protein gene of the cucumber mosaic virus.

EP 0 480 310 A2

This invention relates to a virus-resistant tomato plant as transformed with the cucumber mosaic virus (hereinafter abbreviated as CMV) coat protein (hereinafter abbreviated as CP) gene.

In recent years, attempts have been made to create novel and useful plants by introducing genes providing virus resistance, herbicide resistance, insect resistance and so on into plants using the genetic engineering technology. In particular, various attempts have been made to breed virus-resistant plants by introducing viral CP genes into plants using the genetic engineering technology [Fusao Motoyoshi: Soshiki Baiyo (Tissue Culture), 16, 13-16, 1990].

As examples of the introduction of a plant virus CP gene into tomato plants, there may be mentioned tomato plants with the tobacco mosaic virus (hereinafter abbreviated as TMV) CP gene (R. S. Nelson et al.: BIO/TECHNOLOGY, 6, 403-409, 1988) and the alfalfa mosaic virus CP gene (N. E. Tumer et al.: The EMBO Journal, 6, 1181-1188, 1987) introduced thereinto.

Generally, however, plant body regeneration from cells or tissue fragments carrying a gene introduced thereinto is difficult in tomato cultivars (Fusao Motoyoshi: Soshiki Baiyo, 16, 13-16, 1990), the so-far known transgenic tomato plant creation examples, inclusive of the examples mentioned above, are all limited to those varieties that are readily regenerable.

Meanwhile, the present inventors previously reported that transformants derived from the tobacco cultivar Bright Yellow by introducing thereinto the CP gene of the CMV (strain Y) infecting and causing great damage to a wide variety of plants, inclusive of plants of the families Cucurbitaceae, Solanaceae, Cruciferae and the like, show virus resistance (Japanese Kokai Tokkyo Koho 64-27476).

While the CMV causes great damage to tomato plants as well, there have been no examples of the creation of virus-resistant tomato plant varieties through introduction of the CP gene of the CMV. The advent is desired of a method of breeding tomato plant varieties resistant to the CMV which comprises introducing the CMV CP gene into tomato cultivars.

The present inventors made intensive investigations in an attempt to develop a method of breeding virus-resistant tomato plants and, as a result, found that steps comprising introducing the CMV CP gene into tomato plant protoplasts, embedding the protoplasts in a solid medium containing alginic acid or a salt thereof, adding a liquid medium thereto and culturing the protoplasts enable callus formation, callus growth and plant body regeneration and that the plant bodies express the CP, whereby virus-resistant tomato plants are produced. The present invention has been completed based on these findings.

Thus, the present invention relates to:

(1) A virus-resistant tomato plant as transformed with the coat protein gene of the cucumber mosaic virus strain Y,

(2) A method for producing a virus-resistant tomato plant which comprises

(a) a process of introducing the coat protein gene of the cucumber mosaic virus into the tomato protoplasts,

(b) a process of embedding the protoplasts in a solid medium containing alginic acid or its salt,

(c) a process of culturing the embedded protoplasts in the presence of a liquid medium added to the solid medium, and

(d) a process of regenerating plant bodies from the cultured protoplasts, and

(3) A virus-resistant tomato plant produced by the method according to the above-mentioned (2).

The transgenic tomato plants of the present invention are resistant to any virus infecting tomato plants such as CMV, TMV, potato virus X (PVX), or the like, in particular, to CMV.

The tomato plants to be used in the practice of the invention may be of any cultured or wild variety. The CMV to be used in the practice of the invention also may be of any strain, for example the strain Y, O, D or Q.

The method for producing virus-resistant tomato plants of the present invention is carried out usually in the below-mentioned way of the consecutive processes.

The present invention is usually carried out as follows.

1) Process of isolating tomato protoplasts (protoplast isolation process)

2) Process of introducing the gene into the protoplasts (gene introduction process)

3) Process of embedding the protoplasts in alginic acid (embedding process)

4) Process of culturing the protoplasts (culturing process)

5) Process of regenerating plant bodies (plant body regeneration process)

In the following, the above consecutive processes are explained one by one.

1) Protoplast isolation process

Protoplasts of tomato can easily be obtained from organs and cultured cells of tomato by a method known per se. Specifically, pectinase (i.e. an enzyme that lyzes pectin, which is an intercellular substance), cellulase (i.e. an enzyme that lyzes the cell wall), an osmoticum such as mannitol, sorbitol, glucose or the like, as well as a known plant tissue culture medium and a conventional plant hormone are dissolved in a CPW solution (Table 1), MES buffer or the like. The resulting enzyme solution is adjusted to a pH of about 4.0 to 8.5. In this enzyme solution, the starting material, i.e. organs or cells of tomato is kept standing or shaken at about 10°C to 35°C for about 5 to 48 hours and then thus produced protoplasts are isolated as follows. After above enzyme treatment, the sample solution may be filtered through a 50- to 100-$\mu$m mesh to remove the undigested cell mass and the like, and subjected to centrifugation or density gradient treatment with sucrose, Ficoll or the like according to the per se known method to separate the enzyme solution from the tomato protoplasts, which are then washed with, for example, a CPW solution containing an osmoticum (hereinafter referred to as CPW solution) to yeied purified protoplasts.

Table 1

| Composition of CPW | |
|---|---|
| Ingredient | Concentration (mg/liter) |
| $KH_2PO_4$ | 25.9 |
| $KNO_3$ | 101.1 |
| $CaCl_2 \cdot 2H_2O$ | 1,484.9 |
| $MgSO_4 \cdot 7H_2O$ | 246.5 |
| KI | 0.159 |
| $CuSO_4 \cdot 5H_2O$ | 0.025 |

2) Gene introduction process

Foreign gene coding for the CP of CMV can be introduced into the protoplasts obtained as described above by any per se known method for gene introduction. The method for gene introduction includes the electroporation method, microinjection method, polyethylene glycol method, high pH-high calcium method, liposome method, Agrobacterium-mediated method, laser method, high-frequency wave method, particle gun method, modifications of these, and combinations of two or more of the methods mentioned above. All of those methods are conventional.

Among these methods for gene introduction, the electroporation method, for instance, comprises suspending the protoplasts purified and prepared as described above in an electroporation buffer, together with the plasmid DNA. In this case, the plasmid concentration is in the range of 0.1 $\mu$g/ml to 10 mg/ml and the protoplast concentration is in the range of 1 x $10^2$ to 1 x $10^8$ protoplasts/ml. The poration buffer to be used in this case has a salt concentration of 0 to 1.0 M.

Electric pulses are applied to the above protoplast-DNA mixture. Square waves, decaying waves and alternate current waves may be used as the pulse waves. In the case of square pulse waves, the pulse width is in the range of 1 nsec to 1,000 msec and the field strength is in the range of 1.0 v/cm to 1,000 kv/cm, preferably 0.5 to 2.5 kv/cm. In the case of decaying waves, the time constant is in the range of 1 $\mu$sec to 10 sec and the field strength is in the range of 1.0 v/cm to 1,000 kv/cm.

After pulse application to the above protoplast-DNA mixture, said mixture is allowed to stand at about 0°C to 25°C for 0 (zero) to 60 minutes. (Thus, the mixture may be subjected to the next procedure without standing at about 0°C to 25°C.) After standing, the protoplast-DNA mixture is centrifuged, the supernatant is removed, and the sediment is suspended in the below-mentioned plant tissue culture medium (hereinafter abbreviated as medium) free of calcium chloride containing alginic acid or a salt thereof (about 0.5 to 5.0 w/v% to a total quantity of the suspension, and the suspension is uniformly mixed. This procedure may be repeated 2 to 10 times. The poration buffer is completely replaced with the medium containing alginic acid or a salt thereof. This does not apply to the case where the poration buffer has a salt concentration of 0 (zero), namely it is pure water; in this case 9 to 100 volumes, per volume of pure water, of the medium containing alginic acid or a salt thereof is added directly to said mixture and the whole mixture is subjected to the next procedure. In that case, the protoplast suspension is adjusted to a protoplast concentration of 1 x $10^2$ to 1 x $10^8$/ml of medium.

3) Protoplast embedding process

3

The protoplast suspension obtained as explained above is dropped into the medium containing calcium chloride (about 20 to 100 mM) and an osmoticum (about 0.2 to 0.7μ) using a Komagome pipet or the like.

In the practice of the invention, alginic acid or a salt thereof may be used alone or two or more members of the group consisting of alginic acid and salts thereof may be used in admixture.

The alginic acid salts suited for use in the practice of the invention includes sodium alginate, potassium alginate, lithium alginate and ammonium alginate, among which sodium alginate is preferred. The osmoticum is, for example, sucrose, mannitol, glucose or sorbitol. Preferably, sucrose is used either alone or in combination with one or more other osmotica in a concentration of about 0.2 to 0.7 M. The mixture is kept standing for 30 minutes to 24 hours and then gelated solid body in which tomato protoplasts are embedded in the solid medium are formed in the calcium chloride solution. The gelated solid body is thoroughly washed with a CPW solution or the like.

Usable as the medium are (i.e. the above-mentioned plant tissue culture medium), for example, Murashige-Skoog medium (hereinafter abbreviated as MS), Gamborg's B5 medium (hereinafter abbreviated as B5), Zapata medium, Shahin medium, Kao medium, and modifications of these. However, these media are used after the calcium chloride contained therein is removed.

Such media may further contain one or more of carbon sources, nitrogen sources, inorganic salts, organic substances and so on as desired.

The carbon sources include sugars such as sucrose, glucose, galactose, fructose and maltose, as well as soluble starch. The nitrogen sources include nitrates, ammonium salts and the like. Preferably, the medium should be quite free of any ammonium salt or contain a small amount of ammonium salt. The inorganic salts include known salts containing an element such as phosphorus, potassium, calcium, magnesium, manganese, copper, zinc, molybdenum, boron, iron, cobalt, or nickel e.g.. The organic substances include, among others, inositol, nicotinic acid, pyridoxine hydrochloride, thiamine hydrochloride, calcium pantothenate, folic acid, p-aminobenzoic acid, biotin, choline chloride, riboflavin, ascorbic acid, vitamin A, vitamin $D_3$, vitamin $B_{12}$, other vitamins, sodium pyruvate, citric acid, malic acid, fumaric acid, other organic acids, coconut milk, casein hydrolyzates, yeast extract and other natural substances. In particular, the addition of inositol is favorable for the formation of protoplast colonies and inositol is added to the medium preferably at a ratio of about 0.1 to 8.0 g/liter.

The medium may further contain one or more plant growth regulators, such as auxins and cytokinins as desired. Such auxins include 2,4-dichlorophenoxyacetic acid (hereinafter abbreviated as 2,4-D), 2,4-dichlorophenylacetic acid, indole-3-acetic acid (hereinafter abbreviated as IAA), indole-3-butyric acid (hereinafter abbreviated as IBA), 1-naphthaleneacetic acid (hereinafter abbreviated as NAA), 2-naphthoxyacetic acid, parachlorophenoxyacetic acid, 2,4,5-trichlorophenoxyacetic acid and 1-naphthaleneacetamide. Said cytokinins include 6-benzyladenine (hereinafter abbreviated as BAP), 2-isopentyladenine, 2-isopentenyladenine, kinetin, zeatin, dihydrozeatin, zeatin riboside and diphenylurea. Among them, 2,4-D and NAA, for instance, are preferred auxins, and BAP, for instance, is a preferred cytokinin.

Such auxins are added to the medium generally in an amount of about 0.01 to 20 ppm, and the cytokinins in an amount of about 0.01 to 15 ppm.

An inorganic or organic acid, an alkali, a buffering agent or the like is added to the medium for the purpose of adjusting the pH thereof to about 5.0 to 8.0.

The dropwise addition of alginic acid or a salt thereof into a solution of calcium chloride or the like forms a gelated solid body embedding tomato protoplasts (hereinafter referred to as bead), and this facilitates medium liquid exchange.

The present method using a beads-shaped gelated solid body is advantageous in that protoplast growth is not adversely affected by mechanical shock or by heating, as seen in the agarose embedding method, and is free of shortcomings as found in the liquid culture method, such as the inhibition of growth caused by wastes excreted from cultured cells and the division or survival inhibition due to protoplast coagulation.

4) Protoplast culturing process

Protoplasts are cultured as follows: The protoplast-embedding alginic acid bead which is usually shaped in beads is transferred to a container, such as a plastic petri dish having a diameter of about 3 to 30 cm, or an Erlenmeyer flask having a capacity of about 20 to 1,000 ml, containing about 1 to 300 ml, preferably about 4 to 20 ml, of a liquid culture medium containing carbon sources, plant hormones, etc. as described above (however containing calcium chloride), and is kept standing or shaken. The amount of the culture medium is preferably adjusted so that part of the beads are kept in contact with air. Any known liquid culture medium for plant protoplast culture can be used.

Although culture conditions essentially vary depending upon the medium's status and composition, culture method and other aspects, it is desirable that protoplasts be cultured in the dark initially and that lighting be intensified gradually with protoplast growth. Culture is carried out at about 15 to 35°C, preferably about 25 to 30°C.

The liquid culture medium is renewed every 3 to 14 days, preferably every 5 to 10 days. Concerning the adjustment of osmotic pressure in the culture medium, it is desirable that the concentration of, for example, sucrose, mannitol or glucose, be reduced or increased as appropriate, preferably at a rate of 0.01 to 0.1M every 5 to 10 days.

Transformants advantageously are selected by using the drug resistance as a marker. Usable as the drug resistance gene in that case are antibiotic resistance genes such as the kanamycin, hygromycin, cycloheximide, ampicillin and chloromycetin resistance genes as well as herbicide resistance genes such as the bialaphos resistance gene. The desired transformants can be selected by adding one of the above-mentioned antibiotics or herbicides to the medium liquid, followed by culturing.

After the above-mentioned culture, tomato protoplast-derived calli are formed for the purpose of plant body regeneration.

### 5) Plant body regeneration process

The calli obtained by the above protoplast culture are transferred to a medium that permits shoot regeneration, such as B5 medium or MS medium supplemented with sucrose, glucose or another carbon source at about 0.1 to 10.0 w/v%. Auxins such as NAA, 2,4-D,LAA and IBA, normally at about 0 to 10 ppm, and cytokinins such as BAP, kinetin and zeatin, normally at about 0 to 20 ppm, may be added as plant hormone. Also a medium solidifying agent such as agar, agarose or Gel-rite may also be added. Use of this medium permits shoot regeneration and rooting to produce plant bodies of tomato. In addition to the medium described above, other media known to induce shoots from plant calli can be used as desired.

Culturing is usually carried out under artificial lighting of about 1,000 to 20,000 lux, preferably about 3,000 to 10,000 lux, at about 15 to 35°C, preferably about 25 to 30°C. After the culturing for 2 to 4 weeks the culture medium is preferably replaced by a new culture medium. That is to say, it is preferable that subculture interval be 2 to 4 weeks. Sub-culture is repeated until the regeneration of protoplants is accomplished.

Complete plants are thus regenerated from protoplasts in 10 days to 1 year.

The present invention is hereinafter described in more detail by means of the following examples.

### Example 1 Breeding of transgenic tomato plants as transformed by introduction of the CP gene of the CMV

### 1) Protoplast isolation process

Seeds of tomato plant [varieties: Paresu (Takii Shubyo K.K.) or Baby Tomato (Yamato Noen)] were disinfected by immersing in 70% ethanol for 1 minute and then in 1% sodium hypochlorite aqueous solution for 10 minutes, washed with 3 portions of sterile water, sown into MS agar medium (MS medium shown in Table 2 containing 3 w/v% sucrose and 0.8 w/v% agar), and allowed to germinate at 25°C under illumination (white fluorescent lamp, 2,000 lux, 16 hours per day).

To 1 g of seedling cotyledons (one week after sowing) was added 20 ml of an enzyme aqueous solution (CPW solution containing 0.3 w/v% Macerozyme R-10, 1.0 w/v% cellulase Onozuka R-10 and 0.4 M mannitol, pH 5.8). After cutting the cotyledons to pieces of width of about 1 mm using a surgical knife, the resultant mixture was allowed to stand in the dark at a temperature of 25°C to 30°C for 16 hours for enzyme treatment for protoplast isolation.

After enzyme treatment, the protoplast suspension was filtered through a 100-$\mu$m nylon mesh and a 50-$\mu$m nylon mesh for removing undigested cellular masses and so on. The filtrate was further subjected to centrifugation (500 rpm, 5 minutes) and to a density gradient using 20% sucrose solution, for purifying protoplasts. Finally, protoplasts were prepared by washing with two portions of the CPW solution containing 0.4 M mannitol (hereinafter abbreviated as washing solution).

### 2) Gene introduction process

### Transformation of tomato protoplasts by electroporation

The protoplasts purified in the above manner were suspended in a poration solution (1 ml) [Plant Cell Reports, 5, 57 (1986)] containing 10 $\mu$g of a plasmid derived from the plasmid pCP-NPT containing the kanamycin resistance gene (NPT-II) and CMV (strain Y) coat protein gene (CMV-CP) on one and the same vector by linearization with ApaL1 prepared by the manner according to EP-A-279433. The protoplast density was $5 \times 10^5$/ml. This suspension (800 $\mu$l) was placed in the space (2 mm) between electrodes of a fusion chamber (Shimadzu SSH-C03) ice-cooled (0°C) on a temperature control stage (Shimadzu SSH-T1) and electric pulses (60 $\mu$sec, 0.75 to 1.50 kv/cm) were applied to the suspension using an electric fusion apparatus (Shimadzu somatic hybridizer SSH-1).

### 3) Protoplast embedding process

After electroporation, the protoplasts were maintained at 0°C for 10 minutes and then admixed uniformly with a protoplast culture medium (OFK-1 medium) containing 0.75 w/v% sodium alginate (Wako Pure Chemical Industries) as shown in Table 3. The density was adjusted to 4 to $9 \times 10^4$/ml. The resultant suspension was dropped into 10 ml of OFK-1 medium containing 50 mM calcium chloride placed in a sterilized plastic dish of 9 cm in diameter using a sterilized pipet. Then, the resultant mixture was allowed to stand at 25°C for 6 hours, whereupon spherical solid bodies (hereinafter abbreviated as beads), about 5 mm in diameter, were formed.

### 4) Protoplast culture process and selection of kanamycin-resistant calli

These beads were washed twice with the washing solution, the OFK-2 medium (4 ml) shown in Table 3 was added to each bead, and cultivation was carried out in the dark at 27.5°C.

After 7 days of cultivation, the medium was replaced with OFK-2 medium (4 ml) adjusted to a sucrose concentration of 0.2 M and supplemented with 50 $\mu$g/ml of kanamycin, followed by shaking culture (40 rpm) in the dark at 25°C for selecting kanamycin-resistant calli.

### 5) Plant body regeneration process

After 4 weeks of cultivation, each bead was placed on a shoot regeneration medium (containing 50 $\mu$g/ml of kanamycin) prepared by supplementing MS medium shown in Table 2 with 0.2 mg/liter of IAA, 2.0 mg/liter of zeatin and 5 w/v% of sucrose and solidifying by addition of 0.3 w/v% of Gel-rite (Gellan Gam, Kelco), and the bead was cultured at 25°C under illumination (white fluorescent lamp, about 3,000 lux, 16 hours per day). After 6 weeks of cultivation, 9 colonies, 2 mm or greater in size, were obtained.

The calli grown in the respective beads were transferred to the shoot regeneration medium with the sucrose concentration alone adjusted to 2 w/v%, and cultivation was continued under the same condition as mentioned above, whereupon shoots were regenerated on the calli in the upper part thereof.

These shoots were further grown, then transplanted to a rooting medium (1/3 diluted MS medium containing 0.01 mg/liter IBA, 1 w/v% sucrose and 0.5 w/v% Gelrite, pH 5.8) and cultured under the same conditions, whereby rooting occurred and perfect plant bodies were regenerated.

Table 2

| Murashige-Skoog (MS) medium | |
|---|---|
| Ingredient | Concentration (mg/liter) |
| $KNO_3$ | 1,900 |
| $NH_4NO_3$ | 1,650 |
| $KH_2PO_4$ | 170 |
| $CaCl_2 \cdot 2H_2O$ | 440 |
| $MgSO_4 \cdot 7H_2O$ | 370 |
| $FeSO_4 \cdot 7H_2O$ | 27.8 |
| $Na_2$-EDTA | 37.3 |
| $MnSO_4 \cdot 4H_2O$ | 22.3 |
| $ZnSO_4 \cdot 7H_2O$ | 8.6 |
| $CuSO_4 \cdot 5H_2O$ | 0.025 |
| $CoCl_2 \cdot 6H_2O$ | 0.025 |
| KI | 0.83 |
| $H_3BO_3$ | 6.2 |
| $Na_2MoO_4 \cdot 2H_2O$ | 0.25 |
| Myoinositol | 100 |
| Nicotinic acid | 0.5 |
| Pyridoxine hydrochloride | 0.5 |
| Thiamine hydrochloride | 0.1 - 1.0 |
| Glycine | 2 |
| pH | 5.7 - 5.8 |

Table 3  Protoplast culture medium

| Ingredient | OFK-1 (mg/liter) | OFK-2 (mg/liter) |
|---|---|---|
| $KNO_3$ | 1,900 | 1,900 |
| $CaCl \cdot 2H_2O$ | - | 440 |
| $MgSO_4 \cdot 7H_2O$ | 370 | 370 |
| $KH_2PO_4$ | 170 | 170 |
| $FeSO_4 \cdot 7H_2O$ | 27.8 | 27.8 |
| $Na_2$-EDTA | 37.3 | 37.3 |
| $MnSO_4 \cdot 4H_2O$ | 22.3 | 22.3 |
| $ZnSO_4 \cdot 7H_2O$ | 8.6 | 8.6 |
| $CuSO_4 \cdot 5H_2O$ | 0.025 | 0.025 |
| $CoCl_2 \cdot 6H_2O$ | 0.025 | 0.025 |
| KI | 0.83 | 0.83 |
| $H_3BO_3$ | 6.2 | 6.2 |
| $Na_2MoO_4 \cdot 2H_2O$ | 0.25 | 0.25 |
| Nicotinic acid | 2.5 | 2.5 |
| Thiamine hydrochloride | 10 | 10 |
| Pyridoxine hydrochloride | 1 | 1 |
| Folic acid | 0.5 | 0.5 |
| Biotin | 0.05 | 0.05 |
| Calcium D-pantothenate | 0.5 | 0.5 |
| Choline chloride | 0.1 | 0.1 |
| Glycine | 0.5 | 0.5 |
| Casein hydrolyzate | 150 | 150 |
| L-Cysteine | 1 | 1 |
| Malic acid | 10 | 10 |
| Ascorbic acid | 0.5 | 0.5 |
| Adenine sulfate | 40 | 40 |
| L-Glutamine | 100 | 100 |
| Myoinositol | 4,600 | 4,600 |
| Riboflavin | 0.25 | 0.25 |
| Sucrose | 102,700 (0.3M) | 85,600 (0.25M) |
| NAA | 0.5 | 0.5 |
| 2,4-D | 1.0 | 1.0 |
| BAP | 0.5 | 0.5 |
| pH | 5.7 - 5.8 | 5.7 - 5.8 |

8

Example 2 Northern blotting for detecting the messenger RNA corresponding to the CMV-CP introduced

A 2-gram portion of leaf tissue of the tomato obtained in Example 1 was frozen with liquid nitrogen and disrupted in a mortar. The thus-obtained powder was transferred to 20 ml of guanidine thiocyanate buffer (6 M guanidium thiocyanate, 0.5 w/v% Sarkosyl, 0.1 M 2-mercaptoethanol, 5 mM sodium citrate, pH 7.0) and further disrupted using a Polytron. After filtration through a nylon mesh, the filtrate was transferred to a 50-ml centrifugal tube and centrifuged at 15,000 rpm and 20°C for 15 minutes. The supernatant was layered on 9 ml of 5.7 M cesium chloride aqueous solution in an SRP 28 rotor tube and centrifugation was performed at 28,000 rpm and 20°C for 24 hours. The supernatant was removed, and 400 $\mu$l of sterile distilled water was added to the sediment and, after complete dissolution, the solution was transferred to a 1.5-ml centrifugal tube. Then, 40 $\mu$l of 5 M NaCl and 900 $\mu$l of ethanol were added to said tube and, after mixing, the mixture was placed at a temperature of - 80°C. Ten minutes later, centrifugation was performed at 15,000 rpm for 15 minutes. The supernatant was removed, 50 $\mu$l of 70% ethanol was added to the sediment, the mixture was stirred with a vortex mixer and then centrifuged at 15,000 rpm and 20°C for 15 minutes, and the supernatant was removed. The sediment was dried in vacuo for 5 minutes, 5 $\mu$l of sterile distilled water and 16 $\mu$l of an RNA sample buffer (18.1 w/v% formaldehyde, 58 w/v% formamide, 5.8 w/v% 20 x MOPS[*1], 18.1 w/v% glycerin dye solution[*2]) were added, and the mixture was stirred with a vortex mixer and, after complete dissolution, allowed to stand in a water bath maintained at 65°C for 10 minutes for thermal denaturing of RNA. The denaturation mixture was cooled in ice and subjected to agarose electrophoresis (gel composition: 6 ml of 20 x MOPS, 105 ml of sterile distilled water, 1.4 g of agarose, 6 ml of formaldehyde; electrophoresis buffer: 100 ml of 1 x MOPS, 5 $\mu$l of 10 mg/ml ethidium bromide).

After electrophoresis, RNA in the gel was transferred to a nylon membrane filter (Northern blotting) and the filter was allowed to stand at 80°C for 2 hours for allowing RNA binding to the membrane.

The membrane filter and 5 ml of a hybridization solution (6 x SSC[*3], 5 x Denhardt solution[*4], 0.1 w/v% sodium dodecyl sulfate, 100 $\mu$g/ml denatured salmon sperm DNA) were placed in a polyethylene bag, the bag was sealed, and the whole was maintained at 65°C for 1 hour. Then, 50 $\mu$l of $^{32}$P-labeled CMV-CP cDNA probe prepared by the manner according to EP-A-279433 was added and, after resealing, the whole was maintained at 65°C for 18 hours. The filter was then placed in 100 ml of 6 x SSC and 10-minute washing was repeated twice with shaking at room temperature. The filter was placed in 200 ml of 6 x SSC and 30-minute washing was repeated twice with shaking at 65°C. Without drying, the filter was wrapped in cellophane wrapping film and subjected to autoradiography. As a result, a band was detected in the same electrophoretic position as that of the messenger RNA corresponding to the CMV-CP.

Example 3 Western blotting for detecting the CMV-CP

A 2.0-gram portion of the plant sample obtained in Example 1 was frozen with liquid nitrogen and

*1  20 x MOPS
0.4 M 3-(N-Morpholino)propanesulfonic          acid
0.1 M Sodium    acetate
0.02 M Ethylenediaminetetraacetic          acid (EDTA)
pH 7.0

*2 Glycerin    dye    solution
50% Glycerin
0.1 mg/ml Bromophenol         blue
0.1 mg/ml Xylene    cyanol
1 mM EDTA

*3  6 x SSC
0.9 M NaCl
0.09 M Citric    acid

*4  5 x Denhardt    solution
1% Ficoll    400
1% Polyvinylpyrrolidone
1% Bovine    serum    albumin

disrupted in a mortar, then 2.0 ml of an extraction buffer[*1]was added, and the mixture was stirred and centrifuged at 8,000 rpm for 5 minutes. The sediment was removed, 200 $\mu$l of Laemmli's sample buffer[*2]was added to 200 $\mu$l of the supernatant, and the mixture was stirred with a vortex mixer and then boiled on a boiling water bath for 3 minutes. This sample solution was subjected to 12.5% SDS polyacrylamide gel electrophoresis, and the gel was equilibrated with a transfer buffer[*3]. A nylon membrane (Biodyne, Paul) was equilibrated with the transfer buffer and placed upon the gel, and electroblotting was carried out. After blotting, the nylon membrane was immersed in a blocking solution and treated at 37°C for 1 hour. The blocking solution was washed off with a washing solution[*4], and the membrane was placed in an antibody incubation solution[*5]with an antibody to CMV (anti-CMV antibody) added thereto, and treated at 25°C for 2 hours. After washing with the washing solution, the membrane was placed in the antibody incubation solution with a horseradish peroxidase (HRP)-conjugated secondary antibody added thereto, and treated at 25°C for 2 hours. The secondary antibody was washed off, the membrane was transferred to an HRP color development solution[*6] and color development was allowed to occur for 15 minutes. As a result, a band was detected in the same electrophoretic position as that of CMV coat protein.

(1) Reagent for HRP color development

4-Chloro-1-naphthol 0.3% (w/v methanol)

(2) 60 $\mu$l of cold 30% $H_2O_2$ added to 100 ml of TBS (20 mM Tris, 500 mM NaCl, pH 7.5)

10 ml of (1) and 50 ml of (2) are mixed together just prior to reaction.

## Claims

1.  A virus-resistant tomato plant as transformed with the coat protein gene of the cucumber mosaic virus strain Y.

2.  A method for producing a virus-resistant tomato plant which comprises

    (a) a process of introducing the coat protein gene of the cucumber mosaic virus into the tomato protoplasts,

    (b) a process of embedding the protoplasts in a solid medium containing alginic acid or its salt,

*1 Extraction      buffer

0.03M  Potassium     phosphate     buffer,    pH  7.5

0.4  M  NaCl

10  mM  2-Mercaptoethanol

*2 Laemmli's     sample     buffer

0.25  M  Tris-HCl    (pH  6.8)

2%  SDS

30%  Glycerol

10%  $\beta$-Mercaptoethanol

0.5%  BPB

*3 Transfer     buffer

0.025  M  Tris

0.192  M  Glycine    (pH  8.4)

20%  Methanol

*4 Washing     solution

0.02  M  Tris-HCl

0.5  M  NaCl

0.05  %  Tween   20

*5 Antibody     incubation     solution

0.02  M  Tris-HCl

0.5  M  NaCl

0.05%  Tween   20

1%  Gelatin

*6 HRP  color  development     solution

(c) a process of culturing the embedded protoplasts in the presence of a liquid medium added to the solid medium, and
(d) a process of regenerating plant bodies from the cultured protoplasts.

3. A virus-resistant tomato plant produced by the method according to claim 2.